# EUROPEAN PATENT SPECIFICATION

(11) **EP 4 092 047 B1**
(45) Date of publication and mention of the grant of the patent: **04.06.2025**
(21) Application number: 21740887.1
(22) Date of filing: 05.01.2021
(51) Int. Cl.: C07K 14/55, C07K 1/107, A61K 38/20, A61K 47/60, A61P 31/04, A61P 31/12, A61P 35/00, A61P 37/02

(54) **DISUBSTITUTED PEGYLATED INTERLEUKIN 2, PREPARATION METHOD THEREFOR AND USE THEREOF**
DISUBSTITUIERTES PEGYLIERTES INTERLEUKIN 2, VERFAHREN ZU SEINER HERSTELLUNG UND SEINE VERWENDUNG
INTERLEUKINE 2 PÉGYLÉE DISUBSTITUÉE, SON PROCÉDÉ DE PRÉPARATION ET SON UTILISATION

(30) Priority: 15.01.2020 CN 202010044132; 28.10.2020 CN 202011168335
(43) Date of publication of application: 23.11.2022
(73) Proprietor: Jenkem Technology Co. Ltd. (Tianjin), Tianjin 300462 (CN)
(72) Inventor: WANG, Qingbin, Tianjin 300462 (CN); QI, Jie, Tianjin 300462 (CN); LI, Yu, Tianjin 300462 (CN)
(74) Representative: HGF
(86) International application number: PCT/CN2021/070268
(87) International publication number: WO 2021/143573

(56) References cited:
- WO-A1-2019/226538
- WO-A2-2015/048498
- CN-A- 103 517 718
- CN-A- 104 984 360
- CN-A- 107 106 654
- CN-A- 110 366 421
- CN-A- 111 378 026
- CHARYCH, D.ET AL.: "Modeling the receptor pharmacology, pharmacokinetics, and pharmacodynamics of NKTR-214, a kinetically-controlled interleukin-2 (IL2) receptor agonist for cancer immunotherapy", PLOS ONE, vol. 12, no. 7, 5 July 2017 (2017-07-05), XP055596425, DOI: 10.1371/journal.pone.0179431
- CHARYCH, D.ET AL.: "Modeling the receptor pharmacology, pharmacokinetics, and pharmacodynamics of NKTR-214, a kinetically-controlled interleukin-2 (IL2) receptor agonist for cancer immunotherapy", PLOS ONE, vol. 12, no. 7, 5 July 2017 (2017-07-05), XP055596425, DOI: 10.1371/journal.pone.0179431

## Description

### TECHNICAL FIELD

The present invention belongs to the technical field of pharmaceuticals, and particularly relates to a preparation method for a mixture of PEGylated interleukin 2, which comprises disubstituted PEGylated interleukin 2.

### BACKGROUND

Interleukin-2 (IL-2) is produced when lymphocyte proliferation is stimulated by a mitogen such as phytohemagglutinin (PHA). The IL-2 produced by lymphocytes acts on cells with IL-2 receptors on their surface, causing the cells to proliferate and exert the biological functions thereof. The research on this factor and its receptors has become increasingly intensive recently, and it has been found that the factor plays an important role in cellular immunity, humoral immunity and immunomodulation, and especially its role and clinical significance in tumor immunotherapy has drawn great attention.
IL-2 was approved for the treatment of renal cell carcinoma and metastatic melanoma many years ago, but the application of commercial drugs has been limited mainly due to the following: (1) low effective rate: the effective rate of IL-2 used alone can only reach about 15-20%; (2) two-sided functions of IL-2: a low dose of IL-2 promotes the proliferation of regulatory T cells and causes immunosuppression, and how to balance the two functions remains to be solved; (3) short half-life: the half-life is only a few minutes, and administration at a large dose is a must for sustained effect; (4) severe adverse reaction: the over-activated immune system may attack visceral organs and thus cause organ failure, and the dose window is relatively narrow. In order to improve the therapeutic effect of IL-2 and reduce its adverse reaction, various combined regimens have been tried, such as that of IL-2 and interferon, IL-2 and lymphokine-activated killer (LAK) cell therapy, and IL-2 and chemotherapy, but none of these is satisfactory.

In recent years, studies have found that the addition of chemical modification to the normal IL-2 molecule can maintain the effect of IL-2 while reducing the use concentration and side effects as well. For example, NKTR-214 developed by Nektar has lower side effects and better specificity, and can more efficiently activate the immune system. Specifically, 6 polyethylene glycol (PEG) modifications are added to the IL-2 molecule to form an inactive medicament; after injection into a tumor patient, the 6 PEG modifications will gradually be shed to form the active 2-PEG and 1-PEG.

To address toxicity of IL-2, certain conjugates of IL-2 have also been proposed; for example, CN103517718A discloses a conjugate of an IL-2 moiety with one or more non-peptide water-soluble polymers and a method for preparing the conjugate.

A form of PEGylated IL-2 is designed in the non-patent literature "Relationship of Effective Molecular Size to Systemic Clearance in Rats of Recombinant Interleukin-2 Chemically Modified with Water-soluble Polymers" in which a PEG molecule is conjugated to the primary amine of IL-2 to form a protein heterogeneous mixture containing 1 IL-2 molecule and 4 PEG molecules. This study found that the covalent attachment of the hydrophilic polymer polyethylene glycol can increase the solubility of IL-2 and reduce the plasma clearance, thereby improving its anti-tumor capability WO2019/226538 discloses mixtures of PEG-IL2. In addition, it describes ion exhcange chromatography or reverse-phase chromatography as purification methods for the obtention of the pegylated IL2 variants.

However, in the prior art, there are neither methods for obtaining high-purity disubstituted PEGylated interleukin 2 nor reports about high-purity PEGylated interleukin 2 and uses thereof.

### SUMMARY

The invention is defined in claims 1-14.

The present invention provides a preparation method for the mixture of PEGylated interleukin 2 comprises the following specific steps:
(1) reacting PEG with IL-2 to obtain a crude product of a PEGylated interleukin 2;
(2) performing gel chromatography filtration to remove free interleukin 2 from the crude product;
(3) performing affinity chromatography on a product in the step (2) by means of an α receptor column, and collecting a flow-through peak component and an elution peak component; and
(4) performing ion exchange separation on the flow-through peak component and/or the elution peak component in the step (3), and collecting to obtain components of the mixture of PEGylated interleukin 2.

Preferably, a content of a disubstituted PEGylated interleukin 2 in the components of the mixture of PEGylated interleukin 2 is greater than 60%,further preferably greater than 70%, particularly preferably greater than 90%.

Preferably, the mixture of PEGylated interleukin 2 further comprises a trisubstituted PEGylated interleukin 2 and/or a monosubstituted PEGylated interleukin 2.

Preferably, a content of the trisubstituted PEGylated interleukin 2 in the mixture of PEGylated interleukin 2 is less than 40%, further preferably less than 20%, particularly preferably less than 10%.

Preferably, a content of the monosubstituted PEGylated interleukin 2 in the mixture of PEGylated interleukin 2 is less than 10%, further preferably less than 5%, particularly preferably less than 2%.

Preferably, the PEG is a linear or branched PEG (2-10 arm branched PEG), including linear PEG, double-ended PEG, 2-arm branched PEG, 4-arm branched PEG, 6-arm branched PEG, 8-arm branched PEG, or the like.

Preferably, the PEG has a molecular weight of 1-100 KDa, such as 1-10 KDa (specifically 1, 2, 3, 4, 5, 6, 7, 8, 9 or 10 KDa), 10-50 KDa (specifically 10, 15, 20, 25, 30, 35, 40, 45 or 50 KDa) or 50-100 KDa (specifically 50, 55, 60, 65, 70, 75, 80, 85, 90, 95 or 100 KDa).

Preferably, the interleukin 2 (IL-2) includes a mammalian wild-type interleukin 2 (IL-2) and active variants.

More preferably, the active variants of interleukin 2 (IL-2) include glycosylated variants or non-glycosylated variants; particularly preferably, the non-glycosylated variants of the interleukin include IL-2 phosphorylated variants and IL-2 polypeptide molecule fusion or polymeric variants.

Preferably, the PEG can be linked to the interleukin 2 through a hydrolytic bond, such as an amide bond, a urethane bond, an amine bond, a thioether bond, or an urea bond.

The present invention also provides a preparation method for the disubstituted PEGylated interleukin 2 comprises:
(1) reacting PEG with IL-2 to obtain a crude product of a PEGylated interleukin 2;
(2) performing gel chromatography filtration to remove free interleukin 2 from the crude product;
(3) performing affinity chromatography on a product in the step (2) by means of an α receptor column, and collecting a flow-through peak component and an elution peak component; and
(4) performing ion exchange separation on the flow-through peak component and/or the elution peak component in the step (3), and collecting to obtain components of the disubstituted PEGylated interleukin 2; preferably, the PEG in the step (1) is a polyethylene glycol derivative with a molecular weight of 1-100 KDa; further preferably, the PEG in the step (1) is methoxy polyethylene glycol succinimidyl propionate.

Preferably, the PEG in the step (1) may have a molecular weight of 1-100 KDa, such as 1-10 KDa (specifically 1, 2, 3, 4, 5, 6, 7, 8, 9 or 10 KDa), 10-50 KDa (specifically 10, 15, 20, 25, 30, 35, 40, 45 or 50 KDa) or 50-100 KDa (specifically 50, 55, 60, 65, 70, 75, 80, 85, 90, 95 or 100 KDa). More preferably, the PEG in the step (1) has a molecular weight of 20 KDa.

Preferably, the PEG in the step (1) may be a linear or branched PEG (2-10 arm branched PEG), including linear PEG, double-ended PEG, 2-arm branched PEG, 4-arm branched PEG, 6-arm branched PEG, 8-arm branched PEG, or the like. More preferably, the PEG in the step (1) is a linear single-chain PEG.

PEG in the step (1) is methoxy polyethylene glycol succinimidyl propionate.

In one embodiment of the present invention, the step (1) comprises: adding methoxy polyethylene glycol succinimidyl propionate and IL2 to a buffer and reacting at room temperature for 0.5-5 h, preferably 1-3 h.

Preferably, a flow rate of loading and elution for the gel chromatography filtration in the step (2) is 0.5-1 mL/min, further preferably 0.75 mL/min;
Preferably, the elution buffer for the gel chromatography filtration in the step (2) is an acetic acid solution and a sodium chloride solution, a concentration of the acetic acid solution is 0.1-0.5 M, and a concentration of the sodium chloride solution is 0.1-0.5 M; more preferably, the elution buffer for the gel chromatography filtration is 0.2 M acetic acid solution and 0.2 M the sodium chloride solution.

In one embodiment of the present invention, the step (2) comprises:
equilibrating a gel chromatography system to be stable; loading a sample, wherein the sample is a solution of the crude product of the PEGylated interleukin 2, and a flow rate is controlled at 0.75 mL/min; after the sample loading is completed, eluting with an elution buffer, i.e., 0.2 M HAc and 0.2 M NaCl solution, preferably until UV is stable; and collecting an elution peak, and
removing the free interleukin 2 from the crude product.

Preferably, the step (3) comprises: loading a sample onto the α receptor affinity column, wherein the sample is the product obtained in the step (2); after the sample loading, eluting with an equilibration buffer, preferably until UV is stable, and collecting the flow-through peak; after washing, using an elution buffer instead to elute and collecting the elution peak.

Preferably, a flow rate of loading and elution in the step (3) is 0.1-1 mL/min, preferably 0.45 mL/min;
preferably, the equilibration buffer in the step (3) is a PBS solution with a concentration of 1-10 mM, and the elution buffer is a balanced salt solution with a concentration of 0.1-0.5 M.

More preferably, the chromatographic column used in the receptor affinity chromatography in step (3) is an α receptor affinity column, the equilibration buffer is 5 mM PBS solution, and the elution buffer is 0.2 M sodium acetate solution and 0.2 M sodium chloride solution.

In one embodiment of the present invention, the step (3) comprises: after a 5 mL α receptor affinity column is installed, equilibrating the system until the UV curve is stable; loading a sample, wherein the sample is the product obtained in the step (2), and the flow rate is controlled at 0.45 mL/min; after the sample loading is completed, eluting with the equilibration buffer until UV is stable, and collecting the flow-through peak; after washing, using an elution buffer instead to elute a mixture of the PEGylated IL-2 and collecting the elution peak.

Preferably, the step (4) comprises: loading one or two of the flow-through peak component and the elution peak component in the step (3) through a sample injection loop, using a washing buffer A1 instead, after the sample loading, for elution until UV is stable, and collecting a flow-through peak; after the washing is completed, using an elution buffer B1 instead for elution, and collecting an elution peak; after the collecting is completed, using an elution buffer A2 instead for elution, and collecting an elution peak; after the collecting is completed, using an elution buffer B2 instead for elution, and collecting an elution peak.

Preferably, the elution flow rate for the ion exchange separation in the step (4) is 1-5 mL/min, more preferably 1 mL/min;
In the ion exchange chromatography, the equilibration buffer is a NaAc solution, and the washing buffer A1 is a NaAc solution; the elution buffer B1 is a NaAc solution, and a conductivity is adjusted to 1-5 mS/cm; the elution buffer A2 is a NaAc solution, and the conductivity is adjusted to 5-10 mS/cm; the elution buffer B2 is a NaAc solution.

More preferably, in the ion exchange chromatography, the washing buffer A1 is 5 mM NaAc pH 5.0, the elution buffer B1 is 5 mM NaAc pH 5.0, and the conductivity is adjusted to 3.4 mS/cm; the elution buffer A2 is 5 mM NaAc pH 5.0, and the conductivity is adjusted to 8.64 mS/cm with 1 M NaCl; the elution buffer B2 is 5 mM NaAc pH 5.0 containing 1 M NaCl.

Preferably, the elution peak for the elution buffer A2 is collected in the step (4), so as to obtain the components of the disubstituted PEGylated interleukin 2.

Unless defined otherwise, all technical and scientific terms used in the present invention have the same meaning as commonly understood by those skilled in the art to which the present invention relates, for example:
as used herein, the terms "interleukin 2" and "IL-2" have the same meaning and are used interchangeably, and may be a natural interleukin 2, a recombinant protein (e.g., recombinant human interleukin 2) or a mutant that also has the function of the natural IL-2 (e.g.,
"IL-2-C125A/L18M/L19S" as described in the doctoral dissertation "Recombinant Human Interleukin-2 (IL-2) Mutant Cloning and Expression and Purification in *Pichia Pastoris* System" by Liu Yan), and also include products obtained by tissue culturing, protein synthesis and cell culturing (natural, recombinant cells or mutants), wherein methods for extraction and isolation of the natural, recombinant IL-2 or mutants are well known to those skilled in the art.

Unless otherwise stated, the term "preventing" include reducing the risks of having, contracting or experiencing a disease, disorder, condition or state, the development and/or progression thereof, and/or symptoms thereof.

Unless otherwise stated, the terms "treatment" and "treating" include inhibiting, delaying, moderating, attenuating, limiting, alleviating, or causing the regression of a disease, disorder, condition or state, the development and/or progression thereof, and/or symptoms thereof.

Unless otherwise stated, the terms "comprise" "comprising" and "containing" are intended to represent "open" or "inclusive" language, such that they include the enumerated elements and also allow for the inclusion of additional, unmentioned elements.

### BRIEF DESCRIPTION OF THE DRAWINGS

FIG. 1 shows a chromatogram of PEGylated interleukin 2.
FIG. 2 shows a chromatogram of PEGylated interleukin 2 after separation of IL-2 by gel chromatography filtration.
FIG. 3 shows the curves of p-Stat5 expression levels of the drugs at different time points.
FIG. 4 shows the curves of change in body weight of the animals in each group.
FIG. 5 shows the curves of change in tumor volume.
FIG. 6 shows the curves of change in the proportion of CD4 T cells to T cells.
FIG. 7 shows the curves of change in the proportion of Treg cells to CD4 T cells in tumor tissues.
FIG. 8 shows the curves of change in the proportion of Th cells to CD4 T cells.
FIG. 9 shows the curves of change of CD8 T cells in tumor cells.
FIG. 10 shows the curves of change in the proportion of CD8/Treg in tumor tissues.
FIG. 11 shows the curves of change in double samples of CD8 and PD-1 T cells.

In the figures, G1 is blank control group, G2 is IL-2 group (3 mg/kg), G3 is NKTR-214 group (2 mg/kg), G4 is sample No. 3 group (2 mg/kg), and G5 is sample No. 7 group (2 mg/kg).

The technical solutions in the examples of the present invention will be described clearly and completely below, and it is apparent that the examples described herein are only some examples of the present invention, but not all of them. Based on the examples of the present invention, all other examples obtained by those of ordinary skill in the art without creative work shall fall within the protection scope of the present invention.

### DETAILED DESCRIPTION

The technical solutions in the examples of the present invention will be described clearly and completely below, and it is apparent that the examples described herein are only some examples of the present invention, but not all of them. Based on the examples of the present invention, all other examples obtained by those of ordinary skill in the art without creative work shall fall within the protection scope of the present invention.

### Example 1: PEGylated Interleukin 2

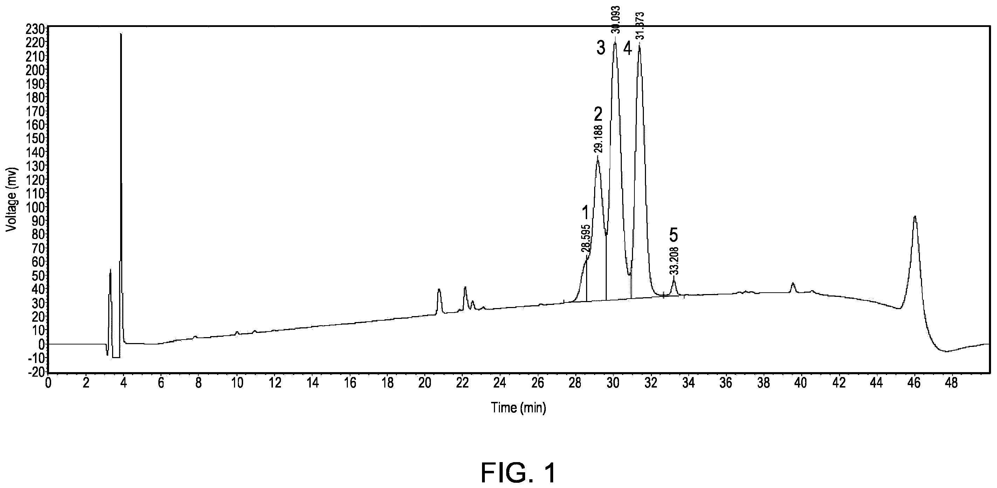

S1: 62.4 mg of methoxy polyethylene glycol succinimidyl propionate with a molecular weight of 20 K (M-SPA-20K) was weighed accurately and added into a 5 mL centrifuge tube;
S2: 1.3 mL of 10 mg/mL IL2 was added, and the resulting mixture was mixed gently until the PEG was completely dissolved;
S3: 3 mL of 50 mM PBS buffer (pH 8.3) was then added, and the resulting mixture was mixed gently and left to stand at room temperature for reaction for 1 h;
S4: the tube was slowly shaken at room temperature for 2 h;
S5: the excess receptors were washed off with 5 column volumes of coupling buffer;
S6: a sample was taken and the content of each component in the reaction mixture was determined by HPLC, and the results are shown in FIG. 1 and Table 1:

**Table 1. Content of each component of PEGylated IL-2**

| **Peak number** | **Components** | **Content (%)** |
|---|---|---|
| 1 | ≥4PEG-IL2 | 2.7 |
| 2 | 3PEG-IL-2 | 20.5 |
| 3 | 2PEG-IL-2 | 34.1 |
| 4 | 1PEG-IL-2 | 31.7 |
| 5 | IL-2 | 11.0 |

### Example 2: Removal of Free Interleukin 2 by Gel Chromatography Filtration

The gel chromatography filtration was set as follows: mobile phase: 5 mM PBS, flow rate: 0.75 mL/min, chromatography column: superdex 200 increase 10/300 GL.

The separation steps were as follows: S1: the chromatography system was equilibrated until the UV curve was stable; S2: the elution peak was collected after the sample obtained in the Example 1 was loaded;

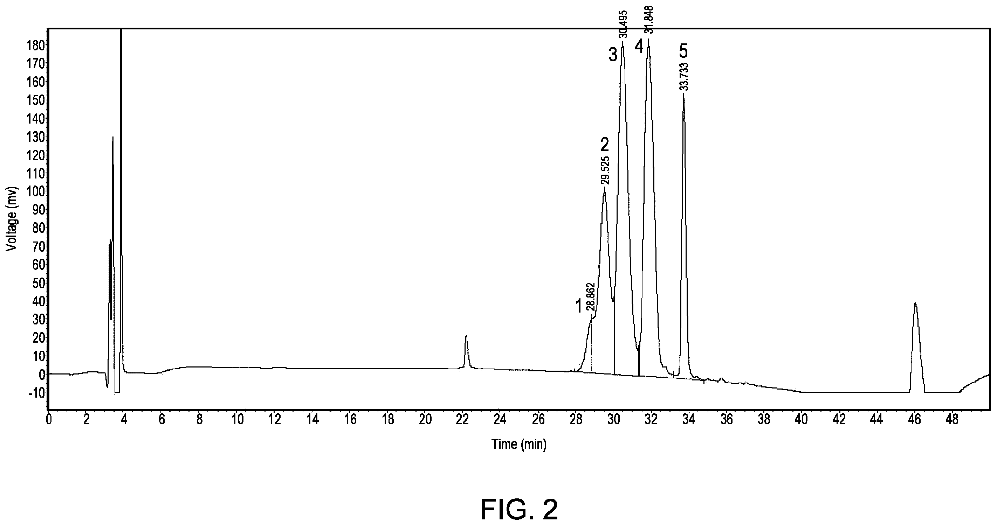

S3: the collected components were concentrated by ultrafiltration and detected by HPLC, and the results are shown in FIG. 2 and Table 2:

**Table 2. Content of each component of PEGylated IL-2 after separation of IL-2 by gel chromatography filtration**

| **Peak number** | **Components** | **Content (%)** |
|---|---|---|
| 1 | ≥ 4PEG-IL2 | 3.2 |
| 2 | 3PEG-IL-2 | 20.5 |
| 3 | 2PEG-IL-2 | 40.1 |
| 4 | 1PEG-IL-2 | 33.8 |
| 5 | IL-2 | 1.0 |

### Example 3: α Receptor Column Affinity Chromatography

The receptor column affinity chromatography was set as follows: mobile phase A: 5 mM PBS, pH 7.0, mobile phase B: 0.2 M HAc + 0.2 M NaCl, flow rate: 0.45 mL/min, column: 5 mL α receptor affinity column, self-made.

The separation steps were as follows: S1: the chromatography system was equilibrated until the UV curve was stable; S2: the flow-through peak and the elution peak were collected after the sample obtained in the Example 2 was loaded.

### Example 4: Separation of Components by Ion Exchange Chromatography

The ion exchange separation chromatography was set as follows: mobile phase A1: 5 mM NaAc pH 5.0, mobile phase B1: 5 mM NaAc pH 5.0, conductivity adjusted to 3.4 mS/cm with 1 M NaCl, mobile phase A2: 5 mM NaAc pH 5.0, conductivity adjusted to 8.64 mS/cm with 1 M NaCl, mobile phase B2: 5 mM NaAc pH 5.0 containing 1 M NaCl, flow rate: 1 mL/min, column: CM FF 1 mL.

The separation steps were as follows: S1: the chromatography system was equilibrated until the UV curve was stable; S2: the flow-through/elution components separated in the Example 3 were each loaded through a sample injection loop; S3: elution was performed with the phase A1 until the UV was stable, and a flow-through peak was collected; S4: elution was performed with the phase B1 instead, and an elution peak was collected; S5: elution was performed with the phase A2 instead, and an elution peak was collected; S6: elution was performed with the phase B2 instead, and an elution peak was collected; S7: the collected components were detected for volume and concentration; S8: the HPLC detection was performed; S9: the collected components were concentrated by ultrafiltration and detected by SDS-PAGE.

### Test Results:

**Table 3. Content of each component of PEGylated IL-2 after separation of the flow-through peak component by ion exchange chromatography**

| No. | Sample Description | ≥4PEG-IL-2 | 3PEG-IL-2 | 2PEG-IL-2 | 1PEG-IL-2 | IL-2 |
|---|---|---|---|---|---|---|
| 1 | Mobile phase A1 elution component | 93.73% | 3.97% | 2.31% | 0 | 0 |
| 2 | Mobile phase B1 elution component | 30.88% | 69.12% | 0 | 0 | 0 |
| 3 | Mobile phase A2 elution component | 0 | 38.14% | 61.86% | 0 | 0 |
| 4 | Mobile phase B2 elution component | 7.21% | 16.45% | 40.18% | 33.66% | 2.50% |

**Table 4. Content of each component of PEGylated IL-2 after separation of the elution peak component by ion exchange chromatography**

| No. | Sample Description | ≥ 4PEG-IL-2 | 3PEG-IL-2 | 2PEG-IL-2 | 1PEG-IL-2 | IL-2 |
|---|---|---|---|---|---|---|
| 5 | Mobile phase A1 elution component | 48.64% | 51.36% | 0 | 0 | 0 |
| 6 | Mobile phase B1 elution component | 0 | 92.39% | 7.61% | 0 | 0 |
| 7 | Mobile phase A2 elution component | 0 | 15.48% | 79.87% | 4.65% | 0 |
| 8 | Mobile phase B2 elution component | 0 | 0 | 28.50% | 71.50% | 0 |

### Example 5: Receptor Affinity Assay

Samples No. 2, No. 3, No. 7 and No. 8 were collected separately, concentrated and then subjected to affinity assay using a SPR system. The receptor proteins are an IL-2α receptor and an IL-2β receptor, and the test results are shown in Table 5. The results showed that the 2PEG-IL-2 prepared by the method described herein generated receptor selectivity and had stronger binding ability to the IL-2β receptor.

**Table 5. Receptor affinity assay for flow-through peak and elution peak**

| Sample Description | Kd(nM) | |
|---|---|---|
| | α receptor | β receptor |
| Sample No. 2 | 7594 | 11850 |
| Sample No. 3 | 2918 | 16980 |
| Sample No. 7 | 1689 | 19120 |
| Sample No. 8 | 563.1 | 4982 |

### Example 6: Flow Cytometry Assay for the Effect of 2PEG-IL-2 on p-Stat5

### 1. Purpose of study

To assess the effect of the test drug 2PEG-IL-2 on the level of p-Stat5 in female C57BL/6 mice at different time points.

### 2. Experimental design

The test compound 2PEG-IL-2 and the positive control drug NKTR214 were administrated separately, blood samples were taken at 0 h, 4 h, 24 h, 48 h, 72 h, 96 h, 120 h, 168 h and 240 h, and the p-Stat5 levels in the blood samples of female C57BL/6 mice were determined by flow cytometry. The specific administration design is shown in Table 6:

**Table 6. Administration design of 2PEG-IL-2**

| Groups | | Concentratio n (mg/kg) | Volume of administration (mL/kg) | Frequency of administration | Number of animals | Test period (days) |
|---|---|---|---|---|---|---|
| 1 | NKTR214 | 0.8 | 10 | Single administration | 36 | 10 |
| 2 | 2PEG-IL-2 | 0.8 | 10 | Single administration | 36 | 10 |

### 3. Experimental materials

3.1 Mouse strain
   Female C57BL/6
3.2 Test drugs
   Test compound 2PEG-IL-2 (sample No. 7 in Example 5)
   Positive control drug NKTR214
3.3 Reagents and antibodies
   Lyse/Fix buffer
   Methanol
   Stain buffer (FBS)
   Hamster Anti-Mouse CD3e-PE
   Phospho-Stat5 (Tyr694) (C71E5) Rabbit monoclonal antibody
   CD16/CD32 monoclonal antibody

### 4. Experimental methods and procedures

4.1Grouping and administration for mice
   4.1.1 The mice were divided into groups of 36 mice by body weight, and the drugs IL-sample 2 and NKTR214 were each administered via tail vein injection at a concentration of 0.8 mg/kg and a volume of 10 mL/kg.
   4.1.2 For each group, four mice were sacrificed by CO₂ at each of 0 h, 4 h, 24 h, 48 h, 72 h, 96 h, 120 h, 168 h and 240 h (where 0 h represents the administration is not performed) and blood was taken from the hearts.
4.2 Sample treatment
   4.2.1 After obtaining samples at each time point, the samples were grouped, and in addition to the administration group of 8 mice, there were also a blank group and a negative group without drug administration.
   4.2.2 200 µL of the obtained mouse blood was pipetted into a centrifuge tube, then 1.8 mL of 1× Lyse/Fix buffer was added, and the mixture was incubated at 37 °C for 10 min. The mixture was centrifuged at 700 g for 5 min and washed once with 500 µL of stain buffer (FBS).
   4.2.3 1 mL of pre-cooled 90% methanol was added, and the mixture was mixed well and placed on ice for 20 min.
   4.2.4 For the blank group, the mixture was centrifuged at 700 g for 5 min and washed once with 500 mL of stain buffer (FBS). Then the mixture was resuspended in 500 µL of stain buffer (FBS), placed on ice for assay later.
   4.2.5 The mixture was centrifuged at 700 g for 5 min and washed twice with 500 mL of stain buffer (FBS). Except for the blank group, the mixtures for other groups were each resuspended in 100 µL of statin buffer (FBS) and added with 1 µL of CD16/32, and the resulting mixture was place on ice for 10 min.
   4.2.6 The mixture was centrifuged at 700 g for 5 min and washed once with 500 mL of stain buffer (FBS). Except for the blank group, the mixtures for other groups were each resuspended in 100 µL of statin buffer (FBS) and added with 1 µL of p-Stat5 and 1 µL of CD3e, and the resulting mixture was incubated at 37 °C for 30 min.
   4.2.7 The mixture was centrifuged at 700 g for 5 min and washed once with 500 mL of stain buffer (FBS). Then the mixture was resuspended in 500 µL of stain buffer (FBS) and used for assay.
   4.3 Data analysis

The expression levels of p-Stat5 cells were analyzed using the analysis software that came with an Attune NxT flow cytometer, and the plotting and data analysis were performed using prism graphpad5.

### 5. Experimental results and discussion

**Table 7. p-Stat5 expression levels of drugs in two groups at different time points**

| | NKTR214 | | | | 2PEG-IL-2 | | | |
|---|---|---|---|---|---|---|---|---|
| | 1 | 2 | 3 | 4 | 1 | 2 | 3 | 4 |
| 0h | 0.037 | 0.019 | 0 | 0.025 | 0.055 | 0.028 | 0.016 | 0.067 |
| 4h | 1.273 | 4.477 | 3.913 | 2.704 | 3.709 | 8.028 | 5.106 | 4.794 |
| 24h | 9.709 | 8.446 | 6.275 | 7.673 | 6.426 | 6.699 | 7.612 | 7.58 |
| 48h | 8.499 | 7.731 | 8.18 | 9.361 | 6.013 | 7.696 | 5.816 | 6.734 |
| 72h | 10.732 | 7.333 | 9.881 | 5.743 | 4.007 | 6.143 | 7.78 | 5.199 |
| 96h | 8.204 | 10.782 | 8.762 | 10.213 | 1.095 | 1.199 | 0.959 | 1.319 |
| 120h | 6.414 | 4.664 | 5.206 | 4.463 | 0.121 | 0.057 | 0.077 | 0.02 |
| 168h | 0.665 | 0.795 | 0.888 | 1.152 | 0.207 | 0.22 | 0.176 | 0.252 |
| 240h | 0.011 | 0.052 | 0 | 0.07 | 0 | 0.02 | 0.025 | 0.078 |

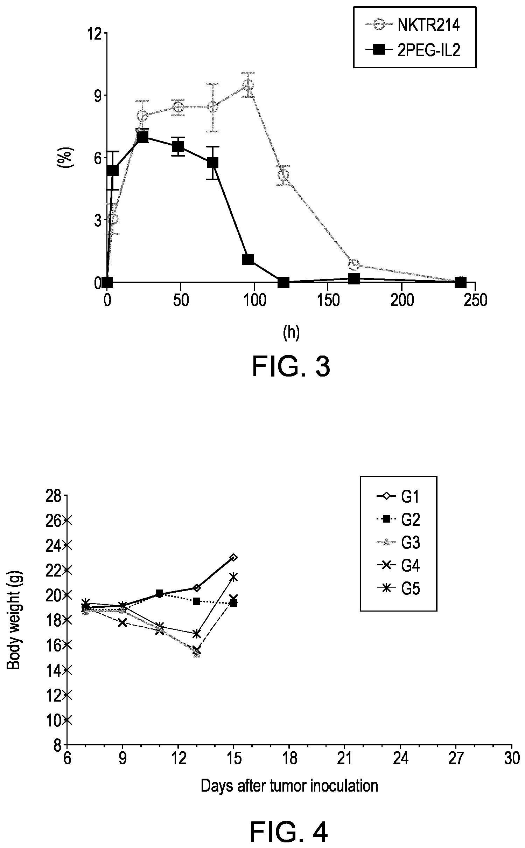

The curves of p-Stat5 expression levels of the drugs in two groups at different time points are shown in FIG. 3.

In this experiment, the drugs IL-sample 2 and NKTR214 were administered via tail vein injection in female C57BL/6 mice, and the p-Stat5 levels at different time points were determined by flow cytometry. The results showed that the area under the curve was smaller for the 2PEG-IL-2 group relative to the NKTR214 group, indicating that it results in lower expression and faster metabolic efficiency of p-Stat5 in mice.

### 6. Experimental conclusion

In this experiment, p-Stat5 expression induced by the test drug 2PEG-IL-2 is metabolized more rapidly relative to the positive drug in the NKTR214 group. The persistence of phosphorylation activation is significantly reduced compared to NKTR214.

### Example 7: Pharmacodynamic Experimental Report of PEG-IL-2

*In vivo* experimental study on anti-tumor effect for B16-F10 subcutaneous tumors in C57BL/6 mice

### 1. Purpose of study

To evaluate the growth of B16-F10 subcutaneous tumors in C57BL/6 mice.

### 2. Experimental design

9 mice/carrier group, 16 mice/treatment group, a total of 5 groups as shown in Table 8:

**Table 8. Mice grouping information**

| Group (G) | Treatment | Mice/group | Frequency of administration | Route of administration |
|---|---|---|---|---|
| 1 | Carrier | 9 | QDx5 | IV |
| 2 | IL-2 | 9+7 | 3mg/kgQDx5 | IV |
| 3 | NKTR-214 | 9+7 | 2mg/kgx1 | IV |
| 4 | Sample No. 3 | 9+7 | 2mg/kgx1 | IV |
| 5 | Sample No. 7 | 9+7 | 2mg/kgx1 | IV |

### 3. Materials:

### 3.1 Animals and living environment

### 3.1.1 Animals

Species: mouse
Strain: C57BL/6 mice
Age: 6-8 weeks
Sex: female
Number of animals: 102 mice
Animal supplier: Shanghai Jihui Biotech Co., Ltd.

### 3.1.2 Living environment

The mice were fed in BioDuro eco-boxes (Shanghai).

The mice were housed in ventilated cages with constant temperature and humidity and there were up to 5 animals in each cage.
Temperature: 20-26 °C.
Humidity: 40-70%.
Cage type: polycarbonate (Suzhou Suhang Technology Equipment Co., Ltd., catalog number: VMU), with a size of 300 mm×180 mm×150 mm. The padding was corn cob (Shanghai Puleteng Biotech Co., Ltd., catalog number: PYMX1), which was changed twice a week.
Diet: during the whole study period, the animals had free access to irradiation-sterilized food in dry granules, and this product is a genuine product for rodents produced by Shanghai Puluteng Biotech Co., Ltd., NO. P1104F.001.
Environmental enrichment: none.
Water: the animals had free access to sterile drinking water.
Cage identification: the identification label for each cage comprised the following information: number of animals, sex, strain and date of receipt, treatment, study number, group number and starting date of treatment.
Animal identification: the animals were ear coded.

### 3.2 Test article

Product identification: IL-2
Physical description: transparent liquid
Packaging and content: 2.63 mg/mL, (2+1) mL
Storage condition: 4 °C
Product identification: NKTR-214
Physical description: transparent liquid
Packaging and content: 0.61 mg/mL, (1+0.82) mL
Storage condition: 4 °C
Product identification: sample No. 3 in Example 5 (receptor flow-through component)
Physical description: transparent liquid
Packaging and content: 0.69 mg/mL, (1+0.51) mL
Storage condition: 4 °C
Product identification: sample No. 7 in Example 5 (receptor elution component)
Physical description: transparent liquid
Packaging and content: 0.57 mg/mL, (1+0.78) mL
Storage condition: 4 °C
Carrier: normal saline

**Table 9. Preparation instructions for the test article formulations for B16-F10 (prepared once daily)**

| Groups | Preparation | Concentration (mg/mL) | Storage |
|---|---|---|---|
| Blank control | Normal saline | - | 4 °C |
| IL-2 | 0.30798 mL of IL-2 was added to 2.39202 mL of normal saline and the mixture was vortexed to obtain a solution. | 2.63 | 4 °C |
| NKTR-214 | 0.7377 mL of NKTR-214 was added to 1.5123 mL of normal saline and the mixture was vortexed to obtain a solution. | 0.61 | 4 °C |
| Sample No. 3 | 0.65217 mL of sample No. 3 was added to 1.59783 mL of normal saline and the mixture was vortexed to obtain a solution. | 0.69 | 4 °C |
| Sample No. 7 | 0.78947 mL of sample No. 7 was added to 1.46053 mL of normal saline and the mixture was vortexed to obtain a solution. | 0.57 | 4 °C |

### 4. Experimental methods and procedures:

### 4.1 Cell culturing

B16F10 was incubated in a 5% CO₂ incubator at 37 °C with RPMI-1640 supplemented with 10% heat-inactivated FBS. The cells were subcultured 3 times a week. The cells had been harvested, counted and subcultured, and inoculated when the confluence was about 70%.

### 4.2 Inoculation of tumors and grouping

When B16f10 reached the desired average size (55-105 mm³), 102 animals were enrolled to the efficacy study. The animals were randomized as follows according to tumor size by using block randomization in Excel. This ensured that all groups were comparable at baseline. 1 × 106 B16F10 cells suspended in 100 µL of PBS had been inoculated subcutaneously into the right flank.

### 4.3 Observation

All procedures related to animal handling, care and treatment in this study were performed according to guidelines approved by Institutional Animal Care and Use Committee (IACUC) of BioDuro and followed the guidelines of The Association for Assessment and Accreditation of Laboratory Animal Care (AAALAC, accreditation number 001516). In routine monitoring, the animals had been examined for any adverse effects of tumor growth and/or treatment on normal behavior, such as motility, food and water consumption (by observation only), and body weight gain/loss (the body weight was measured twice weekly during the pre-administration phase and daily during the administration phase), eye/hair matting, and any other abnormal effects, including tumor ulceration.

### 4.4 Tumor measurements and endpoints

Tumor volume was measured in two dimensions twice weekly with a caliper, and the volume was expressed in mm³ by the following formula: V = 0.5a × b², where a and b are the long and short diameters of the tumor, respectively. The tumor volume was then used to calculate the T-C and T/C values. T-C was calculated, where T is the median time (in days) required for tumors in the treatment group to reach a predetermined size and C is the median time (in days) for tumors in the control group to reach the same size. The T/C value (in percent) is an indication of anti-tumor efficacy; T and C are the average volumes of the treatment and control groups, respectively, on a given day. The T-C value was calculated according to TV. T-C had been calculated with T as the median time (days) required for tumors in the treatment group to reach a predetermined size and C as the median time (days) for tumors in the control group to reach the same size. The tumor tissues were photographed and weighed at the end of the study.

### 4.5 FACS analysis

The tumor tissues were collected at different time points, and after digestion and decomposition of the tumor tissues into individual cells, the cells were subjected to immunofluorescence staining with CD45, CD3, CD4, CD8, CD25 and FOXP3. The stained cells were counted with a flow cytometer and then the proportions of cells with different labels were calculated.

### 5. Results

### 5.1 Body weight

The laboratory animals in groups 3, 4 and 5 showed significant weight loss; there were only 4 animals left in group 4 on day 7 after drug injection, and all of these animals were sacrificed on day 7, so data for day 10 were missing. The curves of change in body weight of the animals in each group are shown in FIG. 4.

### 5.2 Tumor volume

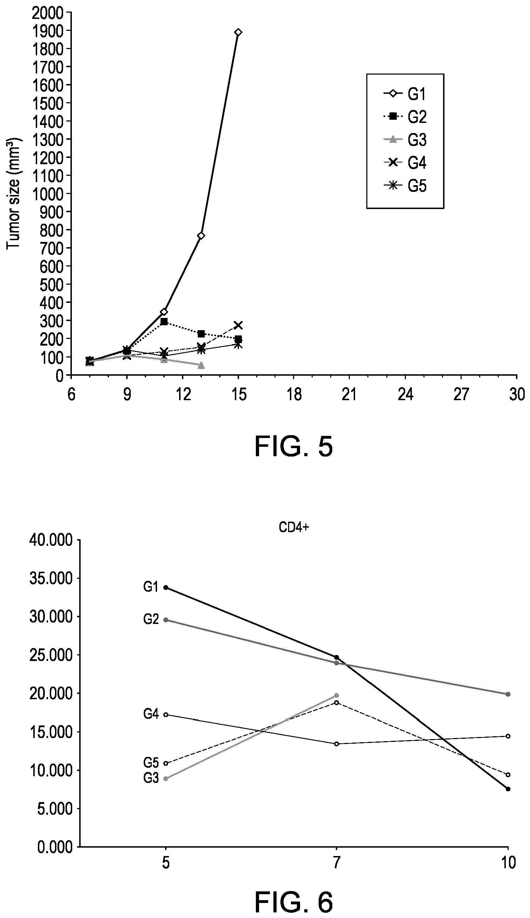

The animals in groups 2, 3, 4 and 5 showed significant anti-tumor activity, and the curves of change in tumor volume are shown in FIG. 5.

### 5.3 CD4 T cells in tumor tissues

The proportions of different immune cells in tumor tissues were determined by FACS, and the curves of change in the proportion of CD4 T cells to T cells are shown in FIG. 6.

### 5.4 Treg cells in tumor tissues

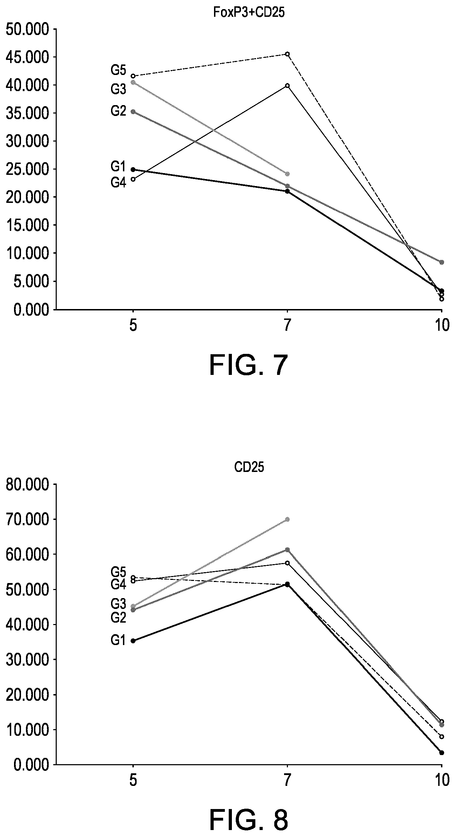

The curves of change in the proportion of Treg cells to CD4 T cells in tumor tissues are shown in FIG. 7.

### 5.5 Th cells in tumor tissues

The curves of change in the proportion of Th cells to CD4 T cells are shown in FIG. 8.

### 5.6 CD8 T cells in tumors

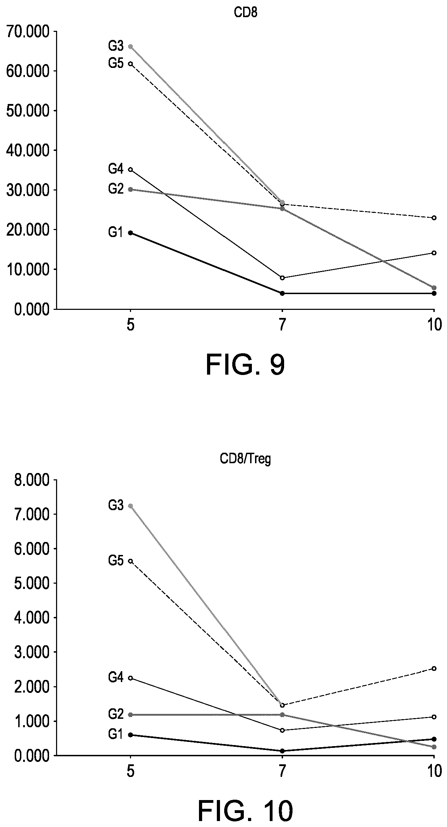

The curves of change of CD8 T cells in tumor cells are shown in FIG. 9.

### 5.7 The proportion of CD8/Treg in tumor tissues

The curves of change in the proportion of CD8/Treg in tumor tissues are shown in FIG. 10.

### 5.8 CD8 and PD-1 T cells in tumor cells

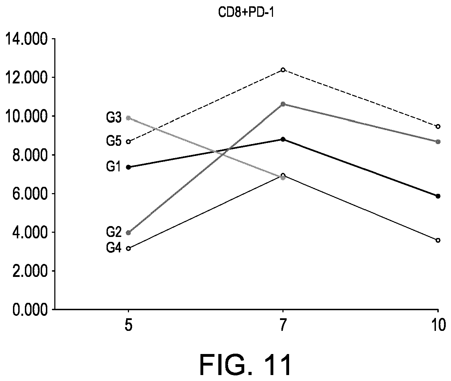

The curves of change in double samples CD8 and PD-1 T cells are shown in FIG. 11.

### 6. Results and discussion

In this experiment, the anti-tumor effect of PEG-IL-2 was tested on B16F10 melanoma-bearing C57BL6 mice, the mechanism of anti-tumor activity of PEG-IL-2 was also studied, and the changes in the content of different types of immune cells in tumor tissues at different stages were tested. The samples include IL2 (group 2) and three PEG-IL-2 components, including NKTR-214 (group 3), sample No. 3 (receptor column flow-through sample) and sample No. 7 (receptor column elution sample).

### 6.1 Toxicity analysis

A stringent toxicity test was not performed in this experiment, but toxicity was observed generally from the body weight changes and deaths of the mice. The body weight changes showed that the body weight of animals in groups 3, 4 and 5 clearly decreased first and then increased. This indicated that all the three PEG-IL-2 were toxic. The animals in NKTR-214 group were almost all dead by the second time point, indicating that NKTR-214 was more toxic at a dose of 2 mg/kg.

### 6.2 Anti-tumor activity

According to the curves of change in tumor volume, IL-2 and three PEG-IL2 showed significant anti-tumor activity, with NKTR-214 showing the minimal tumor volume.

### 6.3 Mechanistic study of PEG-IL-2

IL-2 is an immune cell molecule that achieves the purpose of killing tumor cells by regulating the type and the number of immune cells. The manner in which PEG-IL-2 inhibits tumors was explored by analyzing the number of different immune cells in tumors. In this experiment, the changes in the number of immune cells labeled with CD45, CD3, CD4, CD8, CD25 and FOXP3 were tested by flow cytometry.

It has been reported in the literature that NKTR-214 is an IL-2 coupled with 6 PEGs, and its mechanism is that receptor selectivity can be generated after PEG coupling, and it is more likely to bind to β receptor. The binding of NKTR-214 to β receptor can increase the proliferation of CD8 cells and inhibit the differentiation of Treg cells, which results in a proportion of CD8/Treg being 400 times that for IL-2.

According to the results of this experiment, both NKTR-214 and sample No. 7 showed significant effect of increasing the proportion of CD8/Treg, so it can be seen that the mechanism of anti-tumor killing of 2PEG-IL-2 sample and that of NKTR-214 should be the same.

A hydrolysis process is required for the NKTR-214 product to exert drug efficacy, and 6PEG-IL-2 is not effective unless it is degraded to 2PEG-IL-2 or 1PEG-IL-2. The hydrolysis process is relatively long, and it has been reported in the literature that only 1 PEG can be degraded about every 23 hours at pH 7.4 and 37 °C, and 92 hours are needed for degrading from 6PEG-IL-2 to 2PEG-IL-2. In contrast, the PEG-IL-2 sample of the present invention does not require the degradation process, so the time for it to exert drug efficacy is earlier than that of NKTR-214.

Finally, it should be noted that, the above examples are only used to illustrate the technical solutions of the present invention rather than to limit the same.

## Claims

1. A preparation method for a mixture of PEGylated interleukin 2, comprising the steps of:
(1) reacting PEG with IL-2 to obtain a crude product of a PEGylated interleukin 2;
(2) performing gel chromatography filtration to remove free interleukin 2 from the crude product;
(3) performing affinity chromatography on a product in the step (2) by means of an α receptor column, and collecting a flow-through peak component and an elution peak component; and
(4) performing ion exchange separation on the flow-through peak component and/or the elution peak component in the step (3), and collecting to obtain components of the mixture of PEGylated interleukin 2.

2. The method of claim 1, wherein the mixture of PEGylated interleukin 2 comprises a disubstituted PEGylated interleukin 2; and wherein the disubstituted PEGylated interleukin 2 is present in the mixture of PEGylated interleukin 2 in an amount greater than 60% (w/w), preferably greater than 70% (w/w), particularly preferably greater than 90% (w/w).

3. The method of claim 2 wherein the mixture of PEGylated interleukin 2 further comprises a trisubstituted PEGylated interleukin 2 and/or a monosubstituted PEGylated interleukin 2.

4. The method of claim 2, wherein the trisubstituted PEGylated interleukin 2 in the mixture of PEGylated interleukin 2 is present in an amount less than 40% (w/w), preferably less than 20% (w/w), more preferably less than 10% (w/w).

5. The method of claim 2, wherein the monosubstituted PEGylated interleukin 2 in the mixture of PEGylated interleukin 2 is present in an amount less than 10% (w/w), preferably less than 5% (w/w), more preferably less than 2% (w/w).

6. A preparation method for a disubstituted PEGylated interleukin 2, comprising the steps of:
(1) reacting PEG with IL-2 to obtain a crude product of a PEGylated interleukin 2;
(2) performing gel chromatography filtration to remove free interleukin 2 from the crude product;
(3) performing affinity chromatography on a product in the step (2) by means of an α receptor column, and collecting a flow-through peak component and an elution peak component; and
(4) performing ion exchange separation on the flow-through peak component and/or the elution peak component in the step (3), and collecting to obtain components of the disubstituted PEGylated interleukin 2; preferably, the PEG in the step (1) is a polyethylene glycol derivative with a molecular weight of 1-100 KDa; further preferably, the PEG in the step (1) is methoxy polyethylene glycol succinimidyl propionate.

7. The method of claim 6, wherein a flow rate of loading and elution in the gel chromatography filtration in the step (2) is 0.5-1 mL/min, the elution is elution with a PBS solution with a concentration of is 1-10 mM.

8. The method of claim 6, wherein the step (2) comprises: equilibrating a gel chromatography system; loading a sample, wherein the sample is a solution of the crude product of the PEGylated interleukin 2, and a flow rate is controlled at 0.75 mL/min; after the sample loading is completed, eluting with an elution buffer, i.e., 0.2 M HAc and 0.2 M NaCl solution, collecting an elution peak, and removing the free interleukin 2 from the crude product.

9. The method of claim 6, wherein the step (3) comprises: loading a sample onto the α receptor affinity column, wherein the sample is the product obtained in the step (2); after the sample loading, eluting with an equilibration buffer and collecting the flow-through peak; after washing, using an elution buffer instead to elute a mixture of the PEGylated IL-2 and collecting the elution peak.

10. The method of claim 9, wherein a flow rate of loading and elution in the step (3) is 0.1-1 mL/min, the equilibration buffer is a PBS solution, and the elution buffer is an acetic acid solution and a sodium chloride solution.

11. The method of claim 9, wherein a chromatography column used in the step (3) is a 5 mL α receptor affinity column, a flow rate is controlled at 0.45 mL/min, the equilibration buffer is 5 mM PBS, and the elution buffer is 0.2 M acetic acid solution and 0.2 M sodium chloride solution.

12. The method of claim 6, wherein the step (4) comprises: loading one or two of the flow-through peak component and the elution component in the step (3), using a washing buffer A1 instead, after the sample loading, for elution until UV is stable, and collecting a flow-through peak; after the washing is completed, using an elution buffer B1 instead for elution, and collecting an elution peak; after the collecting is completed, using an elution buffer A2 instead for elution, and collecting an elution peak; after the collecting is completed, using an elution buffer B2 instead for elution, and collecting an elution peak, wherein the washing buffer A1 is a sodium acetate solution, the elution buffer B1 is a sodium acetate solution, and a conductivity is adjusted to 1-5 mS/cm; the elution buffer A2 is a sodium acetate solution, and the conductivity is adjusted to 5-10 mS/cm; the elution buffer B2 is a sodium acetate solution.

13. The method of claim 12, wherein a flow rate of the elution is 1-5 mL/min, the washing buffer A1 is 5 mM NaAc pH 5.0, the elution buffer B1 is 5 mM NaAc pH 5.0, and the conductivity is adjusted to 3.4 mS/cm; the elution buffer A2 is 5 mM NaAc pH 5.0, and the conductivity is adjusted to 8.64 mS/cm with 1 M NaCl; the elution buffer B2 is 5 mM NaAc pH 5.0 containing 1 M NaCl.

14. The method of claim 12 or 13, wherein the elution peak for the elution buffer A2 is collected to obtain the components of the disubstituted PEGylated interleukin 2.

## Patentansprüche

1. Herstellungsverfahren für ein Gemisch von PEGyliertem Interleukin 2, umfassend die folgenden Schritte:
(1) Reagieren von PEG mit IL-2, um ein Rohprodukt eines PEGylierten Interleukins 2 zu erhalten;
(2) Durchführen von Gelchromatographiefiltration, um freies Interleukin 2 aus dem Rohprodukt zu entfernen;
(3) Durchführen von Affinitätschromatographie an einem Produkt in dem Schritt (2) mittels einer α-Rezeptorsäule und Sammeln einer Durchfluss-Peakkomponente und einer Elutions-Peakkomponente; und
(4) Durchführen von Ionenaustauschtrennung an der Durchfluss-Peakkomponente und/oder der Elutions-Peakkomponente in dem Schritt (3) und Sammeln, um Komponenten des Gemischs von PEGyliertem Interleukin 2 zu erhalten.

2. Verfahren nach Anspruch 1, wobei das Gemisch von PEGyliertem Interleukin 2 ein disubstituiertes PEGyliertes Interleukin 2 umfasst; und wobei das disubstituierte PEGylierte Interleukin 2 in dem Gemisch von PEGyliertem Interleukin 2 in einer Menge vorhanden ist, die größer als 60 % (w/w), bevorzugt größer als 70 % (w/w), besonders bevorzugt größer als 90 % (w/w) ist.

3. Verfahren nach Anspruch 2, wobei das Gemisch von PEGyliertem Interleukin 2 ferner ein trisubstituiertes PEGyliertes Interleukin 2 und/oder ein monosubstituiertes PEGyliertes Interleukin 2 umfasst.

4. Verfahren nach Anspruch 2, wobei das trisubstituierte PEGylierte Interleukin 2 in dem Gemisch von PEGyliertem Interleukin 2 in einer Menge vorhanden ist, die weniger als 40 % (w/w), bevorzugt weniger als 20 % (w/w), bevorzugter weniger als 10 % (w/w) ist.

5. Verfahren nach Anspruch 2, wobei das monosubstituierte PEGylierte Interleukin 2 in dem Gemisch von PEGyliertem Interleukin 2 in einer Menge vorhanden ist, die weniger als 10 % (w/w), bevorzugt weniger als 5 % (w/w), bevorzugter weniger als 2 % (w/w) ist.

6. Herstellungsverfahren für ein disubstituiertes PEGyliertes Interleukin 2, umfassend die folgenden Schritte:
(1) Reagieren von PEG mit IL-2, um ein Rohprodukt eines PEGylierten Interleukins 2 zu erhalten;
(2) Durchführen von Gelchromatographiefiltration, um freies Interleukin 2 aus dem Rohprodukt zu entfernen;
(3) Durchführen von Affinitätschromatographie an einem Produkt in dem Schritt (2) mittels einer α-Rezeptorsäule und Sammeln einer Durchfluss-Peakkomponente und einer Elutions-Peakkomponente; und
(4) Durchführen von Ionenaustauschtrennung an der Durchfluss-Peakkomponente und/oder der Elutions-Peakkomponente in dem Schritt (3) und Sammeln, um Komponenten des disubstituierten PEGylierten Interleukins 2 zu erhalten; wobei bevorzugt das PEG in dem Schritt (1) ein Polyethylenglykolderivat mit einem Molekulargewicht von 1-100 KDa ist; ferner bevorzugt das PEG in dem Schritt (1) Methoxypolyethylenglykolsuccinimidylpropionat ist.

7. Verfahren nach Anspruch 6, wobei eine Flussrate der Ladung und Elution in der Gelchromatographiefiltration in dem Schritt (2) 0,5-1 ml/min ist, wobei die Elution Elution mit einer PBS-Lösung mit einer Konzentration von 1-10 mM ist.

8. Verfahren nach Anspruch 6, wobei der Schritt (2) Folgendes umfasst: Äquilibrieren eines Gelchromatographiesystems; Laden einer Probe, wobei die Probe eine Lösung des Rohprodukts des PEGylierten Interleukins 2 ist und eine Flussrate bei 0,75 ml/min gesteuert wird; nachdem die Probenladung abgeschlossen ist, Eluieren mit einem Elutionspuffer, d. h. 0,2 M HAc- und 0,2 M NaCl-Lösung, Sammeln eines Elutionspeaks und Entfernen des freien Interleukins 2 aus dem Rohprodukt.

9. Verfahren nach Anspruch 6, wobei der Schritt (3) Folgendes umfasst: Laden einer Probe auf die α-Rezeptor-Affinitätssäule, wobei die Probe das Produkt ist, das in dem Schritt (2) erhalten wird; nach dem Probenladen Eluieren mit einem Äquilibrierungspuffer und Sammeln des Durchfluss-Peaks; nach dem Waschen Verwenden eines Elutionspuffers stattdessen, um ein Gemisch des PEGylierten IL-2 zu eluieren und Sammeln des Elutions-Peaks.

10. Verfahren nach Anspruch 9, wobei eine Flussrate der Ladung und Elution in dem Schritt (3) 0,1-1 ml/min ist, der Äquilibrierungspuffer eine PBS-Lösung ist und der Elutionspuffer eine Essigsäurelösung und eine Natriumchloridlösung ist.

11. Verfahren nach Anspruch 9, wobei eine Chromatographiesäule, die in dem Schritt (3) verwendet wird, eine α-Rezeptor-Affinitätssäule mit 5 ml ist, eine Flussrate bei 0,45 ml/min gesteuert wird, Äquilibrierungspuffer 5 mM PBS ist und der Elutionspuffer 0,2 M Essigsäurelösung und 0,2 M Natriumchloridlösung ist.

12. Verfahren nach Anspruch 6, wobei der Schritt (4) Folgendes umfasst: Laden von einem oder zwei von der Durchfluss-Peakkomponente und der Elutionskomponente in dem Schritt (3), Verwenden eines Waschpuffers A1 stattdessen nach dem Probenladen zur Elution, bis UV stabil ist, und Sammeln eines Durchfluss-Peaks; nachdem das Waschen abgeschlossen ist, Verwenden eines Elutionspuffers B1 stattdessen zur Elution und Sammeln eines Elutions-Peaks; nachdem das Sammeln abgeschlossen ist, Verwenden eines Elutionspuffers A2 stattdessen zur Elution und Sammeln eines Elutions-Peaks; nachdem das Sammeln abgeschlossen ist, Verwenden eines Elutionspuffers B2 stattdessen zur Elution und Sammeln eines Elutions-Peaks, wobei der Waschpuffer A1 eine Natriumacetatlösung ist, der Elutionspuffer B1 eine Natriumacetatlösung ist und eine Leitfähigkeit auf 1-5 mS/cm eingestellt ist; der Elutionspuffer A2 eine Natriumacetatlösung ist und die Leitfähigkeit auf 5-10 mS/cm eingestellt ist; der Elutionspuffer B2 eine Natriumacetatlösung ist.

13. Verfahren nach Anspruch 12, wobei eine Flussrate der Elution 1-5 ml/min ist, der Waschpuffer A1 5 mM NaAc pH 5,0 ist, der Elutionspuffer B1 5 mM NaAc pH 5,0 ist und die Leitfähigkeit auf 3,4 mS/cm eingestellt ist; der Elutionspuffer A2 5 mM NaAc pH 5,0 ist und die Leitfähigkeit auf 8,64 mS/cm mit 1 M NaCl eingestellt ist; der Elutionspuffer B2 5 mM NaAc pH 5,0 ist, der 1 M NaCl enthält.

14. Verfahren nach Anspruch 12 oder 13, wobei der Elutionspeak für den Elutionspuffer A2 gesammelt wird, um die Komponenten des disubstituierten PEGylierten Interleukins 2 zu erhalten.

## Revendications

1. Procédé de préparation d'un mélange d'interleukine 2 PEGylée, comprenant les étapes de :
(1) mise en réaction du PEG avec une IL-2 pour obtenir un produit brut d'une interleukine 2 PEGylée ;
(2) réalisation d'une filtration par chromatographie sur gel pour éliminer une interleukine 2 libre du produit brut ;
(3) réalisation d'une chromatographie d'affinité sur un produit de l'étape (2) au moyen d'une colonne de récepteur α, et collecte d'un composant de pic d'écoulement et d'un composant de pic d'élution ; et
(4) réalisation d'une séparation par échange d'ions sur le composant de pic d'écoulement et/ou le composant de pic d'élution de l'étape (3), et collecte pour l'obtention de composants du mélange de l'interleukine 2 PEGylée.

2. Procédé de la revendication 1, dans lequel le mélange d'interleukine 2 PEGylée comprend une interleukine 2 PEGylée disubstituée ; et dans lequel l'interleukine 2 PEGylée disubstituée est présente dans le mélange d'interleukine 2 PEGylée en une quantité supérieure à 60 % (p/p), de préférence supérieure à 70 % (p/p), de manière particulièrement préférée supérieure à 90 % (p/p).

3. Procédé de la revendication 2, dans lequel le mélange d'interleukine 2 PEGylée comprend en outre une interleukine 2 PEGylée tri-substituée et/ou une interleukine 2 PEGylée monosubstituée.

4. Procédé de la revendication 2, dans lequel l'interleukine 2 PEGylée tri-substituée dans le mélange d'interleukine 2 PEGylée est présente en une quantité inférieure à 40 % (p/p), de préférence inférieure à 20 % (p/p), idéalement inférieure à 10 % (p/p).

5. Procédé de la revendication 2, dans lequel l'interleukine 2 PEGylée monosubstituée dans le mélange d'interleukine 2 PEGylée est présente en une quantité inférieure à 10 % (p/p), de préférence inférieure à 5 % (p/p), idéalement inférieure à 2 % (p/p).

6. Procédé de préparation d'une interleukine 2 PEGylée disubstituée, comprenant les étapes de :
(1) mise en réaction du PEG avec une IL-2 pour obtenir un produit brut d'une interleukine 2 PEGylée ;
(2) réalisation d'une filtration par chromatographie sur gel pour éliminer une interleukine 2 libre du produit brut ;
(3) réalisation d'une chromatographie d'affinité sur un produit de l'étape (2) au moyen d'une colonne de récepteur α, et collecte d'un composant de pic d'écoulement et d'un composant de pic d'élution ; et
(4) réalisation d'une séparation par échange d'ions sur le composant de pic d'écoulement et/ou le composant de pic d'élution de l'étape (3), et collecte pour l'obtention de composants de l'interleukine 2 PEGylée disubstituée ; de préférence, le PEG de l'étape (1) est un dérivé de polyéthylène glycol présentant un poids moléculaire de 1 à 100 KDa ; en outre de préférence, le PEG de l'étape (1) est le méthoxy polyéthylène glycol-propionate de succinimidyle.

7. Procédé de la revendication 6, dans lequel un débit de chargement et d'élution dans la filtration par chromatographie sur gel à l'étape (2) est de 0,5 à 1 ml/min, l'élution est une élution avec une solution de PBS d'une concentration de 1 à 10 mM.

8. Procédé de la revendication 6, dans lequel l'étape (2) comprend : l'équilibrage d'un système de chromatographie sur gel ; le chargement d'un échantillon, dans lequel l'échantillon est une solution du produit brut de l'interleukine 2 PEGylée, et un débit est régulé à 0,75 ml/min ; une fois le chargement de l'échantillon terminé, l'élution avec un tampon d'élution, c'est-à-dire une solution de HAc 0,2 M et de NaCl 0,2 M, la collecte d'un pic d'élution et l'élimination de l'interleukine 2 libre du produit brut.

9. Procédé de la revendication 6, dans lequel l'étape (3) comprend : le chargement d'un échantillon sur la colonne d'affinité de récepteur α, dans lequel l'échantillon est le produit obtenu à l'étape (2) ; après le chargement de l'échantillon, l'élution avec un tampon d'équilibrage et la collecte du pic d'écoulement ; après le lavage, l'utilisation d'un tampon d'élution à la place pour éluer un mélange de l'IL-2 PEGylée et la collecte du pic d'élution.

10. Procédé de la revendication 9, dans lequel un débit de chargement et d'élution à l'étape (3) est de 0,1 à 1 ml/min, le tampon d'équilibrage est une solution de PBS, et le tampon d'élution est une solution d'acide acétique et une solution de chlorure de sodium.

11. Procédé de la revendication 9, dans lequel une colonne de chromatographie utilisée à l'étape (3) est une colonne d'affinité de récepteur α de 5 ml, un débit est régulé à 0,45 ml/min, le tampon d'équilibrage est du PBS 5 mM, et le tampon d'élution est une solution d'acide acétique 0,2 M et une solution de chlorure de sodium 0,2 M.

12. Procédé de la revendication 6, dans lequel l'étape (4) comprend : le chargement d'un ou de deux composants du pic d'écoulement et du composant d'élution de l'étape (3), l'utilisation d'un tampon de lavage A1 à la place, après le chargement de l'échantillon, pour l'élution jusqu'à ce que les UV soient stables, et la collecte d'un pic d'écoulement ; une fois le lavage terminé, l'utilisation d'un tampon d'élution Bl à la place pour l'élution, et la collecte d'un pic d'élution ; une fois la collecte terminée, l'utilisation d'un tampon d'élution A2 à la place pour l'élution, et la collecte d'un pic d'élution ; une fois la collecte terminée, l'utilisation d'un tampon d'élution B2 à la place pour l'élution, et la collecte d'un pic d'élution, dans lequel le tampon de lavage A1 est une solution d'acétate de sodium, le tampon d'élution B1 est une solution d'acétate de sodium, et une conductivité est ajustée à 1 à 5 mS/cm ; le tampon d'élution A2 est une solution d'acétate de sodium, et la conductivité est ajustée à 5 à 10 mS/cm ; le tampon d'élution B2 est une solution d'acétate de sodium.

13. Procédé de la revendication 12, dans lequel un débit de l'élution est de 1 à 5 ml/min, le tampon de lavage A1 est du NaAc 5 mM à pH 5,0, le tampon d'élution B1 est du NaAc 5 mM à pH 5,0, et la conductivité est ajustée à 3,4 mS/cm ; le tampon d'élution A2 est du NaAc 5 mM à pH 5,0, et la conductivité est ajustée à 8,64 mS/cm avec du NaCl 1 M ; le tampon d'élution B2 est du NaAc 5 mM à pH 5,0 contenant du NaCl 1 M.

14. Procédé de l'une des revendications 12 ou 13, dans lequel le pic d'élution pour le tampon d'élution A2 est collecté pour obtenir les composants de l'interleukine 2 PEGylée disubstituée.
